# EUROPEAN PATENT APPLICATION

(11) **EP 4 810 597 A1**
(43) Date of publication of application: **23.09.2026**
(21) Application number: 24890652.1
(22) Date of filing: 12.11.2024
(51) Int. Cl.: C12M 1/26, C12M 1/36, C12M 1/38, C12M 1/34, C12M 1/00

(54) **SINGLE CELL OPERATING DEVICE AND OPERATING METHOD**

(30) Priority: 13.11.2023 CN 202311498818; 13.11.2023 CN 202311498819
(71) Applicant: SINBODA TECHNOLOGY (ZHEJIANG) CO., LTD., Jiaxing, Zhejiang 314599 (CN)
(72) Inventor: LI, Bing, Jiaxing, Zhejiang 314599 (CN)
(74) Representative: Metida
(86) International application number: PCT/CN2024/131515
(87) International publication number: WO 2025/103297

(57) **Abstract**

Disclosed is a single-cell manipulation device, including a stage, a probe, a probe control assembly, a microscopic imaging assembly, and a pneumatic pressure source assembly. The pneumatic pressure source assembly is configured to provide positive and/or negative pneumatic pressure to the probe in the form of pulses. A method for extracting single cells, a method for injecting a reagent into a single cell and/or extracting contents from a single cell, and a method for performing sample spotting using the single-cell manipulation device are further disclosed. By providing pulsed positive and negative pneumatic pressure to the probe, the present disclosure not only enables precise in-situ extraction of target cells, but also allows the extracted cells to enter the interior of the probe. Thus, the probe serves as a temporary storage site for cells, enabling continuous extraction of multiple cells and improving operational efficiency.

## Description

### TECHNICAL FIELD

This disclosure relates to the field of cell manipulation technology, specifically to a single-cell manipulation device and methods operating the same.

### BACKGROUND

Currently, practical contact-based cell sorting and separation methods are mainly classified into two types: flow extraction and in-situ extraction.

Taking flow extraction as an example, which uses flow cytometry and involves performing specific fluorescent labeling on the cell membranes of target cells in a cell sample. After the sample is dissociated and suspended, the target cells are separated from other cells in a sorting channel based on the presence or absence of a fluorescent signal. This method is suitable for sorting large quantities of cell samples, such as classification and screening of hundreds of thousands or even more cells, but it cannot sort small cell populations, such as a population of only a few thousand cells. Moreover, the accuracy of this method cannot reach 100%. In addition, this method requires prior dissociation treatment of the biological sample and cannot perform real-time in-situ sorting and processing based on dynamic response changes of cells.

In-situ extraction mainly adopts the mode of using a holding pipette to aspirate single cells for spatial position transfer, such as the FLUIDFM flow cytometry system with a microneedle aspiration system. In these systems, the microtube probe used for cell extraction has a front opening of approximately 1-5 µm, and the extraction and release of single cells are achieved through pneumatic pressure changes within the microtube. While this method can achieve 100% accurate in-situ extraction, it can only extract one cell at a time, resulting in low experimental efficiency and inability to achieve continuous extraction of a large number of cells.

### SUMMARY

To address the aforementioned technical problems, this disclosure presents a single-cell manipulation device and methods, which solve the problem of low efficiency in traditional in-situ extraction methods.

Technical solutions

A single-cell manipulation device, including:
a stage, configured to hold a container of the samples;
a probe, which is an elongated hollow structure with opposite front and rear ends;
a probe control assembly, including a probe holder for fixing the probe and a moving platform for moving the probe holder in space to adjust the position of the probe;
a microscopic imaging assembly, providing a viewing window for positioning the probe; and
a pneumatic pressure source assembly, which is in communication with the probe via the holder and supplies positive and/or negative pneumatic pressure to the probe in the form of pulses.

Optionally, the pneumatic pressure source assembly includes a plunger pump, wherein the plunger of the plunger pump moves intermittently within a single working stroke to generate the pulses.

Optionally, the plunger pump provides multiple consecutive pulses within a single working stroke.

Optionally, the pneumatic pressure source assembly includes a solenoid valve, and the plunger pump is connected to the holder via the solenoid valve.

Optionally, the solenoid valve is provided with a common port, a normally closed port, and a normally open port. The plunger pump is provided with ports which are connected to the common port for air inlet and outlet. The normally closed port is connected to the probe holder via a pipeline. When the plunger pump is in operation, the normally open port is closed and the normally closed port is open. When the plunger pump is reset, the normally open port is open and the normally closed port is closed.

Optionally, the normally closed port is connected to a first check valve and a second check valve via a three-way connector, wherein the first check valve and the second check valve have opposite check directions.

Optionally, two sets each of the probe, the probe control assembly and the pneumatic pressure source assembly are provided. The two probes are disposed obliquely toward each other with front ends close to one another.

Optionally, the two probes are a first probe and a second probe, wherein the front-end aperture of the first probe is no greater than 1 µm, and the front-end aperture of the second probe ranges from 5 µm to 20 µm.

Optionally, the pressure value of the pulse ranges from -10 kPa to 10 kPa, and the period is no more than 1 s.

This disclosure provides a method for extracting cells using the aforementioned single-cell manipulation device, including the following steps:
(1) Operating the probe control assembly to move the probe so that a front end thereof approaches the target cell; and
(2) applying, by the pneumatic pressure source assembly, negative pneumatic pressure to the probe to aspirate a single cell, which is the target cell, into the interior of the probe.

Optionally, the single-cell manipulation device includes two probes, a first probe and a second probe, which are connected to an independent pneumatic pressure source assembly. The first probe is pre-filled with a cell dissociation agent, and the second probe is used to extract the target cells. The method further includes the following steps:
operating the probe control assembly to move the first probe so that the front end thereof approaches the target cell; and
applying positive pneumatic pressure to the first probe via the pneumatic pressure source assembly, so as to release the cell dissociation agent around the target cell, thereby bringing the target cell close to or into a dissociated state.

Optionally, the probe is pre-filled with cell culture medium.

Optionally, the method includes transferring the probe, and applying positive pneumatic pressure to the probe via the pneumatic pressure source assembly to eject the target cell.

Optionally, steps (1) and (2) are repeated to aspirate multiple target cells one by one into the interior of the probe.

This disclosure provides a method for injecting reagents into a single cell or extracting contents from a single cell using the aforementioned single-cell manipulation device, including the following steps:
(1) operating the probe control assembly to move the probe so that a front end thereof approaches and pierces the target cell;
(2) applying positive pressure to the probe to inject the reagent into the target cell, or, applying negative pressure to the probe to extract contents from the target cell, via pneumatic pressure source assembly; and
(3) operating the probe control assembly to move the probe so that a front end thereof withdraws from the target cell.

Optionally, the single-cell manipulation device includes two probes, a first probe and a second probe, which are connected to an independent pneumatic pressure source assembly. The first probe is used to inject reagents or extract contents. The method further includes the following steps:
operating the probe control assembly to move the second probe so that the front end thereof approaches the target cell;
applying negative pneumatic pressure to the second probe via the pneumatic pressure source assembly, so that the front end thereof aspirates and holds the target cell.

Optionally, after injection or extraction is completed, the pneumatic pressure source assembly applies positive pressure to the second probe so that a front end thereof detaches from the target cell.

This disclosure provides a method for spotting samples using the single-cell manipulation device, including the following steps:
(1) pre-loading the probe with a sample;
(2) operating the probe control assembly to move the probe so that a front end thereof approaches a spotting position; and
(3) applying positive pneumatic pressure to the probe to drop the sample onto the spotting position.

The present disclosure further provides a single-cell manipulation device, including:
a stage, configured to hold a container of the samples;
a probe, which is an elongated hollow structure with opposite front and rear ends;
a probe control assembly, including a probe holder for fixing the probe and a moving platform for moving the probe holder in space to adjust the position of the probe;
a microscopic imaging assembly, providing a viewing window for positioning the probe; and
a pneumatic pressure source assembly, including a plunger pump and a solenoid valve, wherein the plunger pump is in communication with the probe via the solenoid valve and the holder.

This disclosure further provides a method for extracting adherent cells, including the following steps:
(1) providing two probes, each of which is an elongated hollow structure with opposite front and rear ends, wherein the two probes are a first probe and a second probe, and the first probe is pre-filled with a cell dissociation agent and the second probe is used to extract target cells;
   (1) moving the first probe so that the front end thereof approaches the target cell;
   (2) applying positive pneumatic pressure to the first probe to release the cell dissociation agent around the target cell, thereby bringing the target cell close to or into a dissociated state;
   (3) moving the second probe so that the front end thereof approaches the target cell; and
   (4) applying negative pneumatic pressure to the second probe to aspirate a single cell, which is the target cell, into an interior of the second probe.

This disclosure provides a method for injecting reagents into suspended cells or extracting contents from a suspended cell, including the following steps:
(1) providing two probes, each of which is an elongated hollow structure with opposite front and rear ends, wherein the two probes are a first probe and a second probe, and the first probe is used for injection or extraction, and the second probe is used for aspiration and holding of target cells;
(2) moving the second probe so that the front end thereof approaches the target cell;
(3) applying negative pneumatic pressure to the second probe so that a front end thereof aspirates and holds the target cell;
(4) moving the first probe so that a front end thereof approaches and pierces the target cell; and
(5) applying positive pneumatic pressure to the first probe to inject the reagent into the target cell, or applying negative pneumatic pressure to the first probe to extract the contents from the target cell.

### Beneficial effects

This disclosure provides pulsed positive and negative pneumatic pressure to the probe, which not only enables precise in-situ extraction of target cells, but also allows the extracted cells to enter the interior of the probe, that is, the probe serves as a temporary storage site for cells, thereby enabling continuous extraction of multiple cells, which improves operational efficiency.

### BRIEF DESCRIPTION OF DRAWINGS

FIG. 1a is a schematic structural view of a single-cell manipulation device (with a single probe) of this disclosure;
FIG. 1b is a schematic structural view of a single-cell manipulation device (with dual probes) of this disclosure;
FIG. 2 is a schematic structural view of the stage;
FIG. 3 is a schematic structural view of the probe;
FIG. 4 is a schematic structural view of the moving platform;
FIG. 5 is a schematic structural view of the probe holder;
FIG. 6 is a schematic structural view of the pneumatic pressure source assembly;
FIG. 7 is a schematic diagram illustrating pulse generation by the plunger pump;
FIG. 8 shows a schematic process of the in-situ cell extraction operation in Example 1;
FIG. 9 shows a schematic process of the intracellular injection operation in Example 2;
FIG. 10 shows a schematic process of the intracellular content extraction operation in Example 3;
FIG. 11 shows a schematic process of the nano-spotting operation in Example 4; and
FIG. 12 is the schematic diagram showing the results of nano-spotting in Example 4.

The reference numerals in the figure are as follows:
10. stage; 11. bottom platform; 12. X-direction platform; 13. Y-direction platform; 14. adapter;
20. microscopic imaging assembly; 21. optical microscope objective lens; 22. image detection unit; 23. light source;
30. probe; 31. front end; 32. rear end;
40. probe holder; 41. main body; 42. fixing nut; 43. sealing rubber ring; 44. vent post;
50. moving platform; 51. X-direction moving base; 52. Y-direction moving base; 53. Z-direction moving base;
60. pneumatic pressure source assembly; 61. plunger pump (61a. inlet port; 61b. outlet port; 63c, plunger); 62. solenoid valve (62a. common port; 62b.normally open port; 62c. normally closed port); 63. three-way connector; 64. first check valve; 65. second check valve; 66. air filter; 67. silencer; 68. plug;
70. control system.

### DESCRIPTION OF EMBODIMENTS

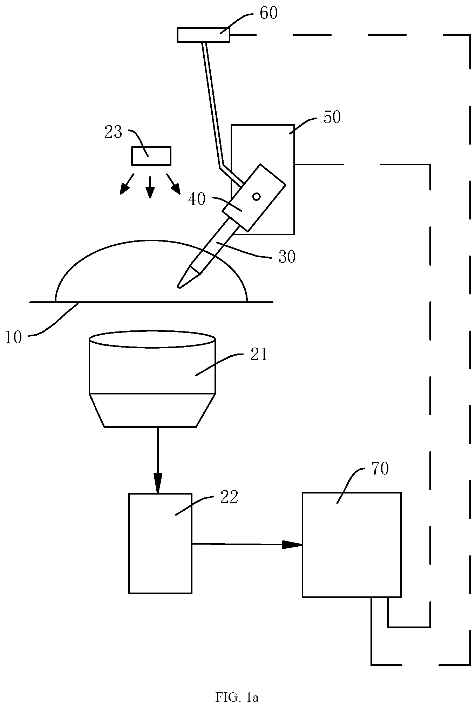

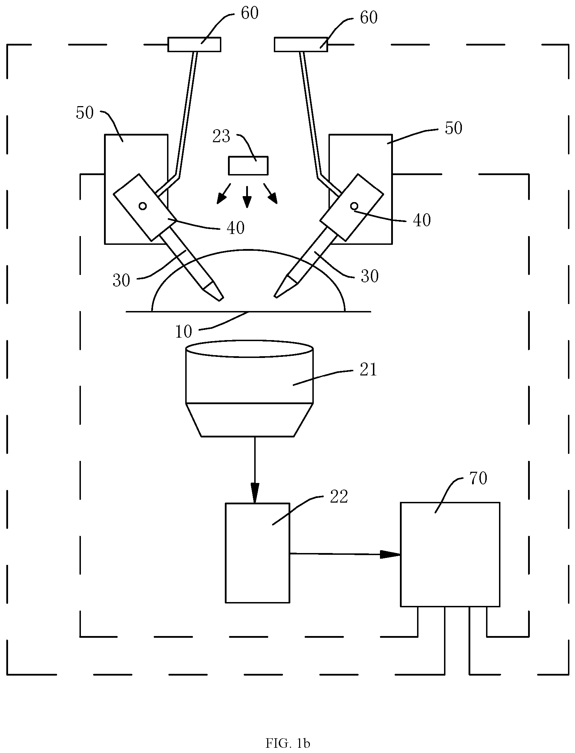

As shown in FIG. 1, a single-cell manipulation device is used for cell-level operations such as cell extraction, injection of reagents into cells, extraction of cellular contents, and biological spotting. The device includes components such as a stage 10, a microscopic imaging assembly 20, a probe 30, and a pneumatic pressure source assembly 60.

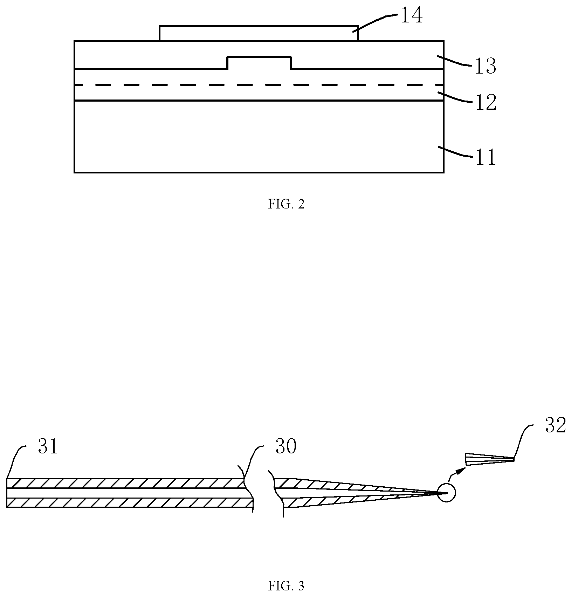

As shown in FIG. 2, the stage 10, serving as the operating platform for holding cell containers, includes a bottom platform 11, an X-direction platform 12, and a Y-direction platform 13. The bottom platform 11 is rotatable about a vertical axis. The X-direction platform 12 is slidably disposed on the bottom platform 11 and can slide horizontally along the X-direction. The Y-direction platform 13 is slidably disposed on the X-direction platform and can move horizontally along the Y-direction. The X and Y directions are perpendicular to each other. The Y-direction platform is equipped with an adapter 14 for holding the cell containers. Each of the bottom platform 11 and the X-direction platform is provided with a through hole corresponding to the adapter 14, facilitating imaging by the microscopic imaging assembly from below the bottom platform 11. The X-direction platform 12 and the Y-direction platform 13 are driven by motors, with a lead screw mechanism arranged between each motor and the respective platform.

As shown in FIG. 3, probe 30 is an elongated hollow structure made of materials such as quartz or glass. Probe 30 may be fabricated by laser heating and pulling of a quartz capillary, to obtain a bare microstructure with geometric rotational symmetry, without movable parts or embedded circuits inside the hollow structure. Probe 30 has opposite front end 32 and rear end 31, both of which are open structures. The size of the rear end 31 is adapted to the probe holder, while the aperture of the front end 32 is correspondingly reduced, providing a flat micro-manipulation end face, so as to avoid damage to cells.

The inner diameter of the front end may be adjusted according to application requirements. When the probe 30 is used to extract cells, the front-end aperture is generally 1.5 to 2 times the cell diameter, approximately 15 to 20 µm. When the probe 30 is used to aspirate and hold the cells, the inner diameter of its front end needs to be smaller than the cell diameter, generally ranging from 5 to 10 µm, which is hereinafter also referred to as a micron-scale hollow quartz probe. When the probe 30 is used to pierce the cell to extract intracellular contents or inject reagents, the front-end aperture is generally less than 1 µm, preferably less than 200 nm, which is hereinafter also referred to as a nano-scale hollow quartz probe.

The number of probes 30 can be adjusted as required, and may be one or two. When the device is used to extract the suspended cells or spot samples onto a support, only a single probe 30 is needed, as shown in FIG. 1a. When the device is used for extracting adherent cells, two probes 30 are required. One probe is used to release the dissociation agent, and the other probe is used to extract cells. When the device is used to inject reagents into suspended cells or extract intracellular contents, one probe is used to aspirate and hold the cells, and the other probe performs reagent injection and content extraction.

When dual probes are adopted, as shown in FIG. 1b, the two probes 30 are arranged to incline toward each other with front ends close to each other, respectively forming an angle of 60° to 80° with the stage 10. Each probe 30 is connected to an independent pneumatic pressure source assembly 60.

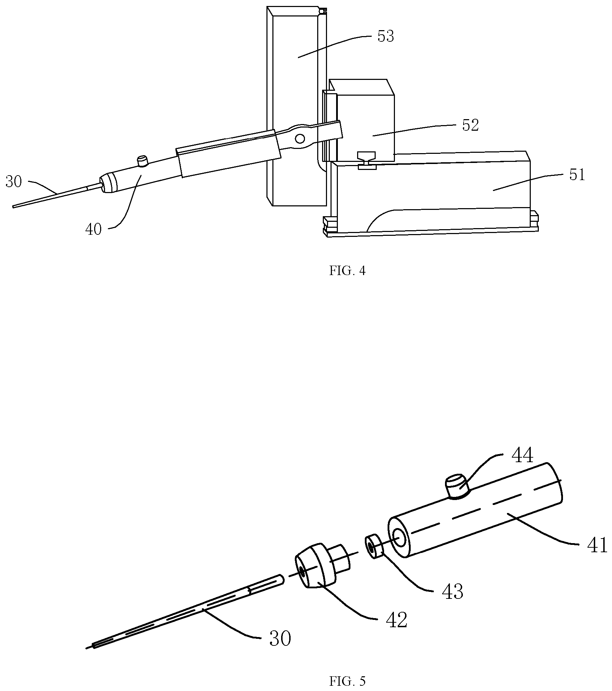

As shown in FIG. 4, the device further includes a probe control assembly, which includes a probe holder 40 and a moving platform 50 for moving the probe holder in space. The moving platform 50 includes an X-direction moving base 51, a Y-direction moving base 52, and a Z-direction moving base 53. The probe holder is fixed to the X-direction moving base 51. The moving platform 50 enables three-dimensional spatial movement of the probe holder and the probe 30, so that the front end 32 of the probe 30 can approach the target cell. Each of the X-direction moving base 51, the Y-direction moving base 52, and the Z-direction moving base 53 is driven by a motor, and a lead screw mechanism is provided between each motor and the corresponding moving base.

As shown in FIG. 5, the probe holder includes a tubular main body 41, a fixing nut 42, and a sealing rubber ring 43. A vent post 44 is provided on the side surface of the main body 41, and the vent post 44 is connected to the pneumatic pressure source assembly 60 through a pipeline. The main body 41 is open at its forward end and closed at its rearward end. The forward end thereof is provided with a threaded hole for threaded connection with the fixing nut 42, and the sealing rubber ring is disposed at the bottom of the threaded hole.

The fixing nut 42 and the main body 41 are each provided with a concentric central hole engaged with the probe 30. The central hole of the fixing nut 42 extends through both ends thereof, and the central hole of the main body 41 is in communication with the vent post 44. The rear end of the probe 30 can pass through the fixing nut 42 and extend into the central hole of the main body 41. When the fixing nut 42 is tightened toward the main body, the sealing rubber ring 43 is compressed and elastically deformed to hold the probe 30. When the fixing nut 42 and the main body 41 are separated, the sealing rubber ring 43 rebounds to release the probe 30, which can then be detached from the probe holder.

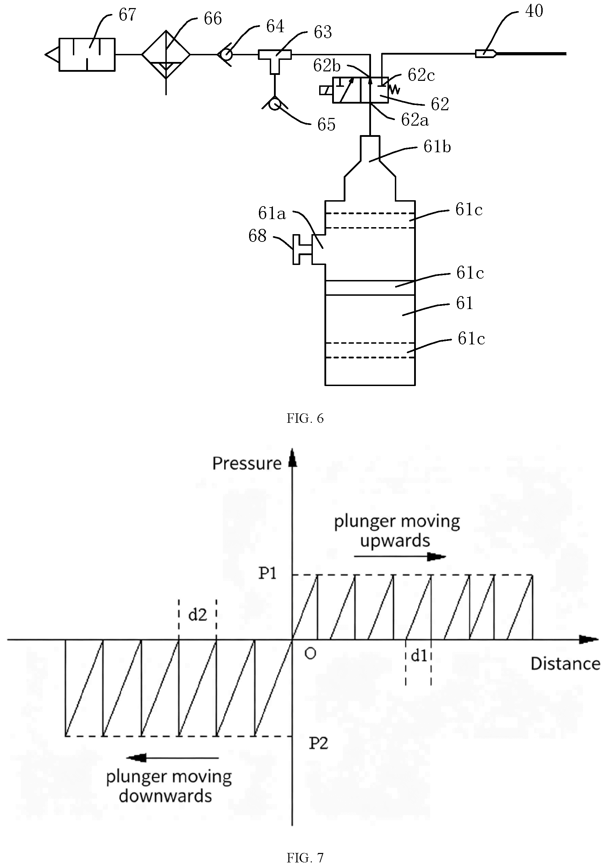

The pneumatic pressure source assembly 60 serves as a power source for aspirating and ejecting substances (cells, reagents, etc.). In some embodiments, as shown in FIG. 6, the pneumatic pressure source assembly includes a plunger pump 61, a solenoid valve 62, a three-way connector 63, a first check valve 64, a second check valve 65, an air filter 66, a silencer 67, and a plug 68. The plunger pump 61 has an inlet port 61a, an outlet port 61b, and a plunger 61c. The solenoid valve 62 has a common port 62a, a normally open port 62b, and a normally closed port 62c. The air filter 66 has an air inlet port and an air outlet port. The first check valve 65 has an "IN" port and an "OUT" port; the second check valve 66 has an "IN" port and an "OUT" port, and the three-way connector 63 has three connection ports.

The plug 68 is screwed into the inlet port 61a of the plunger pump, tightened, and sealed. The intake and exhaust of the plunger pump are both implemented through the outlet port 61b. The outlet port 61b of the plunger pump is connected to the common port 62a of the solenoid valve 62 by a pipeline. The inlet port of the air filter 66 is connected and sealed to the silencer 67 by a threaded connection. The outlet port of the air filter 66 is connected to the "IN" port of the first check valve 64 by a pipeline. The "OUT" port of the first check valve 64 is connected to one of the ports of the three-way connector 63 by a pipeline. Among the other two ports of the three-way connector 63, one port is connected to the normally open port 62b of the solenoid valve 62 by a pipeline, and the other port is connected to the "IN" port of the second check valve 65 by a pipeline. The normally closed port 62c of the solenoid valve 62 is connected to the probe holder by a pipeline and further communicated with the probe 30, so that the entire pneumatic pressure source assembly 60 is in communication with the probe 30.

A silencer 67 is provided at the inlet port of the air filter 66, to reduce noise of the entire pneumatic circuit system for cell operation. The outlet port of the air filter 66 is connected to the "IN" port of the first check valve 64. The first check valve 64 prevents backflow of gas in the entire pneumatic circuit system into the air filter 66. The second check valve 65 is used to expel air from the plunger pump 61, ensuring no unfiltered air remains in the plunger pump 61.

Before operation, the common port 62a and normally open port 62b of solenoid valve 62 are communicated first, while the normally closed port 62c is closed. The plunger 61c first moves upwards to the top dead center, during which residual gas inside the plunger pump 61 is exhausted through the three-way connector 63 and the second check valve 65. Thereafter, the plunger 61c moves downwards to the mid-stroke position of the plunger pump 61. During this process, air passes through the air filter 66 and enters the plunger pump 61 through the first check valve 64 and the three-way connector 63. The initial resting position of the plunger 61c is set at the mid-stroke position of the plunger pump, allowing it to move downward to apply negative pneumatic pressure or upward to apply positive pneumatic pressure.

In some embodiments, as shown in FIG.7, the plunger pump 61 applies positive or negative pneumatic pressure to the probe 30 in the form of pulses, and the plunger 61c moves intermittently within a single working stroke. When the plunger 61c moves upward intermittently, each movement distance being d1, multiple positive pneumatic pressure pulses can be generated, each having a pressure value of P1 and a pulse period of T1; and when the plunger 61c moves downward intermittently, with each movement distance being d2, multiple negative pneumatic pressure pulses can be generated, each having a pneumatic pressure value of P2 and a pulse period of T2.

In the figure, the origin O indicates that plunger 61c is in its initial position, where the pneumatic pressure inside the plunger is approximately equal to atmospheric pressure. Generally, the pulse pneumatic pressure value is selected in the range of -10 kPa to 10 kPa, with a pulse period of less than 1 s, preferably 0.5 s to 1 s. The pulse pneumatic pressure value and period are adjustable to meet different practical needs.

The working principle of the pneumatic pressure source assembly is described below taking cell extraction as an example:

The solenoid valve 62 is energized, its normally open port 62b is closed, and its normally closed port 62c is opened, thereby bringing the plunger pump 61 into communication with the probe 30. The plunger 61c inside the plunger pump 61 is controlled to move downward, thereby providing negative pneumatic pressure pulses to the probe 30 via the plunger pump 61, allowing the probe 30 to aspirate cells from the culture dish. If a single pulse is insufficient to aspirate a cell into the interior of the probe 30, consecutive pulses can be applied until aspiration is achieved. During cell aspiration, when the internal and external pneumatic pressures reach equilibrium, the cells cease movement and can thus be retained inside the probe 30.

After the cell aspiration is completed, if the plunger 61c has not yet reached its limit position, it can continue to move to provide pulses so as to aspirate more cells.

When plunger 61c moves downward to the limit position, the normally closed port 62c of solenoid valve 62 is closed, the normally open port 62b is connected, and the plunger 61c then moves upward for resetting. During the resetting process, the gas inside plunger pump 61 is expelled through three-way connector 63 and second check valve 65. After the plunger 61c completes resetting, the normally open port 62b is closed and the normally closed port 62c is opened, allowing for continued cell aspiration. By repeating this cycle, more cells from different positions can be aspirated into the probe 30 in multiple batches.

After cell aspiration is complete, the plunger 62 is first reset (or may not be reset). The plunger 61c inside plunger pump 61 is controlled to move upward, thereby providing positive pneumatic pressure pulses to the probe 30, allowing the probe 30 to eject cells into the culture dish. Since the positive pneumatic pressure is also provided in the form of pulses, cells can be ejected one by one until all cells are ejected. Its working process is the reverse of the cell aspiration process, and will not be described in detail here.

By using positive or negative pneumatic pressure pulses, at most one cell can be ejected or aspirated per operation. When a single negative pneumatic pressure pulse is applied, the aspiration force may be insufficient to aspirate a cell into the interior of the probe. Applying a single negative pneumatic pressure pulse may result in insufficient aspiration, preventing the cell from entering the probe. By continuously delivering multiple pulses, cells can eventually be aspirated into the probe. The same principle applies when ejecting cells.

Due to the relatively large interior space of the probe, which can temporarily store a considerable number of cells, the probe 30 can continuously aspirate multiple cells and then eject them all in one action, significantly improving operational efficiency. In addition, by using pulses, cells can be ejected by the probe 30 one by one, allowing them to be placed at different positions and eliminating the need for redistribution.

As can be seen, the solenoid valve 62 serves as a pressure balancing device for the entire system. When the normally open port 62b is closed and the normally closed port 62c is open, the plunger pump 61 does not work, and the pneumatic circuit system communicating the probe 30 with the plunger pump 61 can maintain pressure balance. The extracted cells can be completely retained in the probe 30, and the plunger 61c can then be moved to generate new pulses. When the normally open port 62b is open and the normally closed port 62c is closed, since pressure balance has already been achieved, the plunger pump 61 performs a reset operation, which does not affect the cells retained in the probe 30.

The microscopic imaging assembly 20 includes a light source 23, an optical microscope objective lens 21, and an image detection unit 22 (such as a CCD camera). The light source is generally disposed directly above the stage 10, and the optical microscope objective lens 21 and the image detection unit 22 are arranged below the stage. The control system 70 may be a terminal device such as a computer. The plunger pump 61 and the solenoid valve 62 in the pneumatic pressure source assembly 60, as well as the motors in the moving platform 40 and the stage 10, are all controlled by the control system. The control system is further provided with an imaging display unit, that is, the probe 30 and target cells can be displayed on a screen via the imaging system, allowing an operator to perform real-time operation.

As described above, the device of the present disclosure can be applied to operations such as cell extraction, intracellular content extraction, injection of reagents into cells, and sample spotting, which will be described one by one hereinafter.

### Example 1 In-situ extraction of adherent or suspension cells

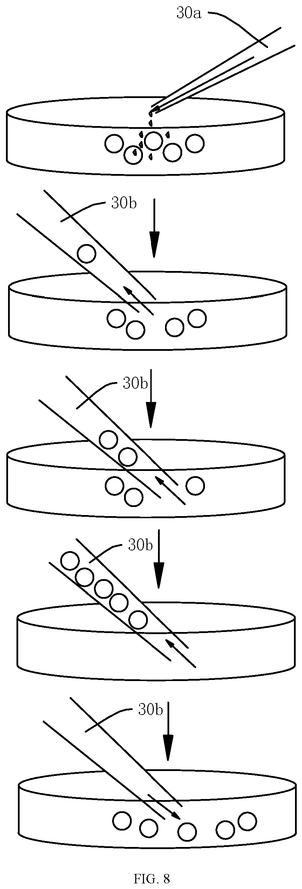

1) The target cells are observed in real time using the microscopic imaging assembly. The cells are then extracted and transferred to a new environment, and the specific process is shown in FIG. 8.
   The target cells are positioned by microscopic imaging based on bright-field features or fluorescence signals.
2) If the target cells are adherent cells, a dual-probe device is required. First, a nano-scale hollow quartz probe 30a with a front-end aperture less than 200 nm is pre-filled with a cell dissociation solution. The cell dissociation solution may be trypsin or ethylenediaminetetraacetic acid, etc.

The probe control assembly is operated to position the front end of the nano-scale hollow quartz probe 30a at a distance of 3-5 µm from a target cell via bright-field imaging. A positive pneumatic pressure pulse in the range of 1-5 kPa is then applied to precisely spray the dissociation solution inside the probe to the periphery of the target cell until the target cell dissociates from its original position. Preferably, the degree of cell dissociation reaches 80% or more, more preferably 90% or more. The duration of the entire process ranges generally from 10 s to 30 s depending on cell type.

If the target cells are suspension cells, the foregoing dissociation step is not required.

3) After dissociation, the nano-scale hollow quartz probe 30a is removed. The front end of the micron-scale hollow quartz probe 30b is positioned 2-3 µm away from the target cell in the bright-field imaging field. Then, a negative pneumatic pressure pulse of -5 kPa with a period of 0.5-1 s is applied to the micron-scale hollow quartz probe 30b via the pneumatic pressure source assembly 60, to aspirate the target cell into the interior of the micron-scale hollow quartz probe 30b.

If a single negative pneumatic pressure pulse fails to aspirate a cell into the micron-scale hollow quartz probe 30b, multiple negative pneumatic pressure pulses may be applied continuously until the step is completed. The pneumatic pressure magnitude and period may also be adjusted. If more target cells need to be aspirated, the above steps may be repeated to aspirate the target cells into the micron-scale hollow quartz probe 30b one by one.

4) After cell aspiration is completed, the two probes are lifted to a safe area, and the stage 10 is moved to position a new cell preservation container, which may be a culture dish, well plate, or EP tube. The micron-scale hollow quartz probe 30b containing the target cells is lowered to the extraction position, the plunger pump is reset, and then a 3 kPa positive pneumatic pressure pulse is applied to eject the target cells from the probe and transfer them to a new environment. Cell ejection may also be performed in multiple batches.

### Example 2 Precise intracellular injection for single cells

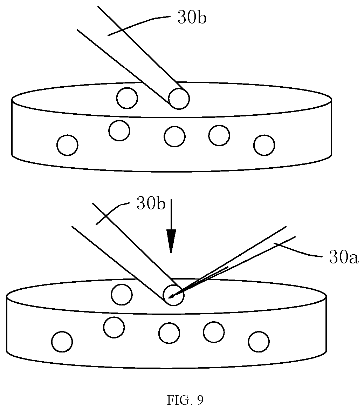

1) The target cells are observed in real time using a microscopic imaging assembly, and precise intracellular injection is performed on the target cells, as shown in FIG. 9.
   The target cells are positioned by a microscopic imaging assembly based on bright-field features or fluorescent signals.
2) If the cells to be injected are suspension cells, a dual-probe device is required. First, the probe control assembly is operated to position the front end of the micron-scale hollow quartz probe 30b with a front-end aperture of 5 µm to contact the target cell membrane via bright-field imaging. A negative pneumatic pressure pulse within the range of 1-3 kPa is applied to the hollow quartz probe 30b via the pneumatic pressure source assembly 60, so as to hold the target cell at the front end of the micron-scale hollow quartz probe 30b. In this operation, the front-end aperture of the micron-scale hollow quartz probe 30b needs to be smaller than the cell diameter, and is generally 5-10 µm. If a single negative pneumatic pressure pulse fails to hold the cell, multiple pulses may be applied continuously until holding is completed.
   If the cells to be injected are adherent cells, this holding step is not required.
3) A nano-scale hollow quartz probe 30a with a front-end aperture less than 200 nm is used as the injection needle. The solution to be injected, such as dye, protein, mRNA, or plasmid, is pre-filled inside the nano-scale hollow quartz probe 30a. The front end of the probe is positioned to contact the target cell membrane through bright-field imaging. Under the operation of the moving platform 40, the front end of the nano-scale hollow quartz probe 30a further advances by 1-3 µm to pierce the cell membrane. A positive pneumatic pressure pulse of 5 kPa is applied to the nano-scale hollow quartz probe 30a via the pneumatic pressure source assembly 60, so as to deliver the injection material inside the probe into the cell. Multiple pulses may be applied continuously until the injection is completed.
4) After injection, the nano-scale hollow quartz probe 30a is retracted by the same distance as it advanced during injection. For suspended cells, a positive pneumatic pressure pulse is applied to the micron-scale hollow quartz probe 30b holding the cell, so as to release the target cell.

If multiple cells need to be injected, the above steps can be repeated.

### Example 3 Precise intracellular content extraction for single cells

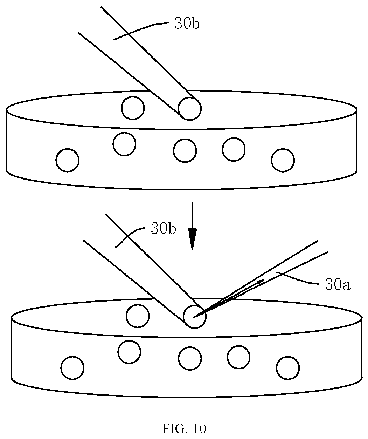

1) The target cells are observed in real time using a microscopic imaging assembly, and the precision intracellular content extraction is performed on the target cells, as shown in FIG. 10.
   The target cells are positioned using microscopic imaging based on bright-field features or fluorescent signals.
2) If the cells to be injected are suspension cells, a dual-probe device is used. First, the probe control assembly is operated to position the front end of the micron-scale hollow quartz probe 30b with a front-end aperture of 5 µm to contact the target cell membrane via bright-field imaging. A negative pneumatic pressure within the range of 1-3 kPa is applied to the micron-scale hollow quartz probe 30b via the pneumatic pressure source assembly 60, so as to hold the target cell to the front end of the probe. If the cells to be extracted are adherent cells, this holding step may be omitted.
3) A nano-scale hollow quartz probe 30a with a front-end aperture of less than 1 µm is used as the extraction needle, the front end of the nano-scale hollow quartz probe 30a is positioned to contact the target cell membrane through bright-field imaging. Driven by the moving platform, the front end of the nano-scale hollow quartz probe 30a further advances by 1-3 µm to pierce the cell membrane. A negative pneumatic pressure pulse of 3-5 kPa is applied to the nano-scale hollow quartz probe 30a via the pneumatic pressure source assembly 60, so as to aspirate intracellular substances into the probe. Multiple pulses may also be applied to aspirate more contents.
4) After extraction, the nano-scale hollow quartz probe 30a is retracted by the same distance as it advanced during injection. For suspended cells, a positive pneumatic pressure pulse may be applied to the micron-scale hollow quartz probe 30b holding the cell, so as to release the target cell.
5) After the intracellular material is aspirated, the nano-scale hollow quartz probe 30a is lifted to a safe area. The stage is moved to position a new storage container, which may be a culture dish, well plate or EP tube. The nano-scale hollow quartz probe 30a containing intracellular material is lowered to the extraction position, and a 3 kPa positive pneumatic pressure pulse is applied to eject the extracted intracellular material from the probe and transfer it to a new environment.

### Example 4 Surface nano-dpotting

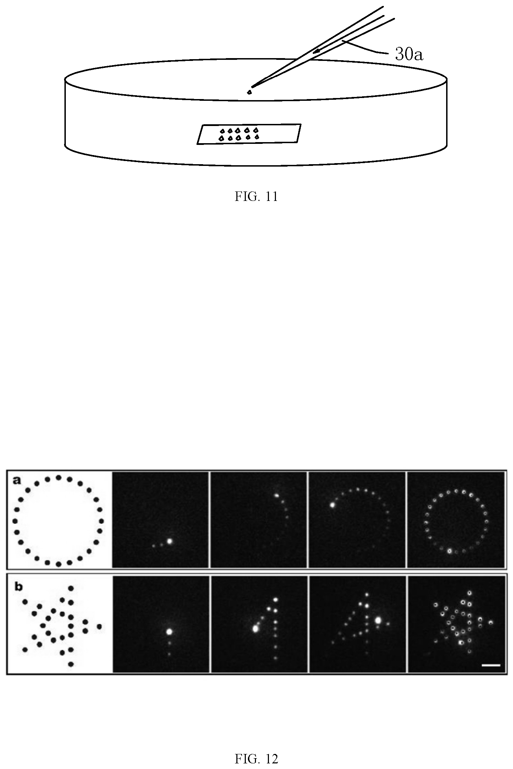

The substrate to be sampled is observed in real time under the microscopic imaging assembly, and nano-spotting is performed on the surface of the substrate, as shown in FIG. 11.
1) A nano-scale hollow quartz probe 30a with a front-end aperture smaller than 1 µm is used as a spotting needle. A solution to be spotted, such as dye, protein, mRNA and the like, is loaded into the nano-scale hollow quartz probe 30a.
2) A target spotting position is located by bright-field microscopic imaging. The front end is positioned 2-3 µm above the spotting position through bright-field imaging. A positive pneumatic pressure pulse of 3-5 kPa is applied to the probe via the pneumatic pressure source assembly 60, causing the spotting solution inside the probe to be dripped onto the substrate surface as discrete droplets. This step may be repeated if multiple spotting operations are required. The final spotting result is shown in FIG. 12.

## Claims

1. A single-cell manipulation device, comprising:
a stage, configured to hold a container of samples;
a probe, being an elongated hollow structure with opposing front and rear ends;
a probe control assembly, comprising a probe holder for fixing the probe and a moving platform for moving the probe holder in space to adjust a position of the probe;
a microscopic imaging assembly, providing a viewing window for positioning the probe; and
a pneumatic pressure source assembly, in communication with the probe via the holder and supplying positive and/or negative pneumatic pressure to the probe in the form of pulses.

2. The single-cell manipulation device according to claim 1, wherein the pneumatic pressure source assembly comprises a plunger pump, and a plunger of the plunger pump intermittently moves within a single working stroke to generate the pulses.

3. The single-cell manipulation device according to claim 2, wherein the plunger pump provides a plurality of continuous pulses within a single working stroke.

4. The single-cell manipulation device according to claim 2, wherein the pneumatic pressure source assembly comprises a solenoid valve, and the plunger pump is connected to the holder via the solenoid valve.

5. The single-cell manipulation device according to claim 4, wherein the solenoid valve has a common port, a normally closed port and a normally open port, the plunger pump has a port for air inlet and outlet connected to the common port, and the normally closed port is connected to the probe holder through a pipeline, wherein when the plunger pump is in operation, the normally open port is closed and the normally closed port is open, and when the plunger pump is reset, the normally open port is open and the normally closed port is closed.

6. The single-cell manipulation device according to claim 5, wherein the normally closed port is connected to a first check valve and a second check valve via a three-way connector, and the first check valve and the second check valve have opposite check directions.

7. The single-cell manipulation device according to claim 1, wherein the probe, the probe control assembly and the pneumatic pressure source assembly are each provided in duplicate, and two probes are inclined toward each other with front ends thereof close to each other.

8. The single-cell manipulation device according to claim 7, wherein the two probes comprise a first probe and a second probe, wherein an aperture of the front end of the first probe is no greater than 1 µm, and an aperture of the front end of the second probe is in a range of 5 to 20 µm.

9. The single-cell manipulation device according to claim 1, wherein each of the pulses has a pneumatic pressure value ranging from -10 kPa to 10 kPa, with a period of no more than 1 second.

10. A method for extracting cells using the single-cell manipulation device according to any one of claims 1-9, comprising the following steps:
(1) operating the probe control assembly to move the probe so that a front end thereof approaches a target cell; and
(2) applying, by the pneumatic pressure source, negative pneumatic pressure to the probe to aspirate a single cell, which is the target cell, into an interior of the probe.

11. The method according to claim 10, wherein the single-cell manipulation device comprises a first probe and a second probe, each being connected to an independent pneumatic pressure source assembly, wherein the first probe is pre-filled with a cell dissociation agent, and the second probe is used to extract the target cell, and the method further comprises the following steps:
operating the probe control assembly to move the first probe so that the front end thereof approaches the target cell; and
applying, by the corresponding pneumatic pressure source assembly, positive pneumatic pressure to the first probe to release the dissociation agent around the target cell, as as to bring the target cell close to or into a dissociated state.

12. The method according to claim 10, wherein the probe is pre-filled with cell culture medium.

13. The method according to claim 10, comprising transferring the probe and applying positive pneumatic pressure to the probe via the pneumatic pressure source assembly to eject the target cell.

14. The method according to claim 10, comprising repeating steps (1) and (2) to aspirate a plurality of target cells into the interior of the probe one by one.

15. A method for injecting a reagent into a single cell or extracting contents from a single cell using the single-cell manipulation device according to any one of claims 1-9, comprising the following steps:
(1) operating the probe control assembly to move the probe so that a front end thereof approaches and pierces the target cell;
(2) applying positive pneumatic pressure to the probe to inject the reagent into the target cell, or applying negative pneumatic pressure to the probe to extract contents from the target cell, via the pneumatic pressure source assembly;
(3) operating the probe control assembly to move the probe so that a front end thereof withdraws from the target cell.

16. The method according to claim 15, wherein the single-cell manipulation device comprises a first probe and a second probe, each being connected to an independent pneumatic pressure source assembly, wherein the first probe is configured to inject reagents or extract contents, and the method further comprises the following steps:
operating the probe control assembly to move the second probe so that the front end thereof approaches the target cell;
applying negative pneumatic pressure to the second probe, so that the front end thereof aspirates and holds the target cell.

17. The method according to claim 16, comprising, after injection or extraction is completed, applying positive pneumatic pressure to the second probe via the corresponding pneumatic pressure source assembly, causing the front end thereof to detach from the target cell.

18. A method for spotting samples using the single-cell manipulation device according to any one of claims 1-9, comprising the following steps:
(1) pre-loading the probe with a sample;
(2) operating the probe control assembly to move the probe so that a front end thereof approaches a spotting position; and
(3) applying, by the pneumatic pressure source, positive pneumatic pressure to the probe to drop the sample onto the spotting position.
